# EUROPÄISCHE PATENTSCHRIFT

(11) **EP 0 854 738 B1**
(45) Veröffentlichungstag und Bekanntmachung des Hinweises auf die Patenterteilung: **14.11.2001**
(21) Anmeldenummer: 96937978.3
(22) Anmeldetag: 06.08.1996
(51) Int. Cl.: A61M 16/06

(54) **VERPACKTE BEATMUNGSMASKE**
PACKAGED BREATHING MASK
MASQUE DE RESPIRATION ARTIFICIELLE EMBALLE

(30) Priorität: 09.08.1995 DE 19529322
(43) Veröffentlichungstag der Anmeldung: 29.07.1998
(73) Patentinhaber: Feuchtgruber, Gottfried, 80337 München (DE)
(72) Erfinder: Feuchtgruber, Gottfried, 80337 München (DE)
(74) Vertreter: Schmidt, Christian, Dipl.-Phys.
(86) Internationale Anmeldenummer: DE9601509
(87) Internationale Veröffentlichungsnummer: WO9705919

(56) Entgegenhaltungen:
- EP-A- 0 350 914
- WO-A-93/16747
- DE-A- 2 244 887
- DE-A- 4 344 403
- US-A- 5 121 745

## Beschreibung

Die Erfindung betrifft eine verpackte Beatmungsmaske, die es dem Anwender ermöglicht, einen Patienten ohne Kontakt zu Haut, Schleimhaut oder Körpersekreten Mund-zu-Mund, Mund-zu-Nase oder mit einem Beatmungsbeutel zu beatmen.

Aus der US-A-5 121 745 ist eine verpackte Beatmungsmaske bekannt, die durch Volumenzunahme von einem kleineren Verpackungsvolumen in ihre endgültige Gebrauchsform bringbar ist. Diese bekannte Beatmungsmaske besteht aus einem flexiblen und formstabilen Kunststoffdom, an dessen einem Außenrand ein aufblasbarer Wulst angeordnet ist, der aus Kunststoff besteht. In ihrem verpackten Zustand ist diese Beatmungsmaske in einem Behälter aufgenommen, der zumindest genauso groß ist, wie der Querschnitt der Beatmungsmaske. Um diese bekannte Beatmungsmaske in ihre endgültige Gebrauchsform zu bringen, soll beim Öffnen eines Ventils Umgebungsluft in die evakuierte Blase 12 strömen und dadurch die Maske aufrichten.

Grundsätzlich sind Beatmungsmasken in verschiedensten Bereichen der Medizin, insbesondere in der Anästhesie und Notfallmedizin bekannt. Diese Masken decken normalerweise Mund und Nasenbereich ab und bestehen aus elastischem Material. Die Masken werden von Hand leicht angepreßt oder von Bändern gehalten. Derartige Masken sind aus der GB-PS 841 104, DE 42 12 259 C1, DE 43 12 215 C1 oder aus der US 3 538 913 bekannt.

In Wiederbelebungssituationen wird vom Ersthelfer, wenn keine entsprechenden medizinischen Gerätschaften vor Ort sind, zur Aufrechterhaltung der Oxygenierung die Mund-zu-Mund oder Mund-zu-Nase-Beatmung gefordert. Dabei kann es durch den Körperkontakt zur Übertragung von Infektionskrankheiten kommen. Vom Fachpersonal wird die Beatmung bis zum Einführen eines Beatmungsschlauches in die Luftröhre mittels einer Mund und Nase umschließenden, glockenförmigen und nach außen durch einen Wulst abgedichteten Maske und einem daran angeschlossenen komprimierbaren Ballon mit Sauerstoffzuleitung durchgeführt.

Für den Ersthelfer werden zum Schutz vor Kontamination bislang verschiedene Hilfen angeboten. Diese zeichnen sich entweder durch zu sperriges Packvolumen oder durch unzureichenden Schutz vor Kontamination (Bedecken von Mund und Nase durch eine Folie) aus. Einige müssen mit einem Mundstück tief in den Mund des Patienten eingeführt werden (DE-GM 87 09 867).

Aus der DE-OS 22 44 887 ist eine Atemmaske aus Integralschaum bekannt, in deren Innerem eine Plexiglasversteifung verläuft.

Es ist das der Erfindung zugrundeliegende Problem (Aufgabe), eine Beatmungsmaske der eingangs genannten Art dahingehend weiterzubilden, daß diese einfacher hergestellt werden kann und in komprimiertem Zustand ein kleineres Packungsvolumen einnimmt.

Die Lösung dieser Aufgabe erfolgt durch die Merkmale des Patentanspruchs 1.

Durch die erfindungsgemäße Lösung ist eine Maske geschaffen, die im ungebrauchten Zustand sehr klein verpackt ist (Schlüsselanhängerprinzip) und gleichzeitig leicht und schnell zu handhaben ist. Die Maske beherbergt keine Verletzungsgefahr für den Patienten und gleicht im gebrauchsfertigen Zustand einer normalen Beatmungsmaske. Somit kann sie zum einen nach vom Ersthelfer begonnener Wiederbelebung vom Fachpersonal bis zur Einführung eines Beatmungsschlauches weiterverwendet werden. Andererseits kann die erfindungsgemäße Maske in Notfallausrüstungen, die klein verpackt sein müssen (Bergwacht, Hubschrauber, Expeditionen, ...), die bislang bekannten und relativ sperrigen Masken ersetzen.

Vorteilhafte Ausführungsformen der Erfindung sind in den Unteransprüchen beschrieben.

Das Gehäuse kann in den Ausmaßen etwa einer Filmpatrone entsprechen. Der Schaumstoffkörper kann mittels Vakuum komprimiert sein und in Folie eingeschweißt sein. Durch Brechen des Vakuums läßt sich die Maske in Sekundenschnelle in die Gebrauchsform bringen. Denkbar ist auch ein Entstehen und Aushärten der Maskenform durch Aufschäumen zweier flüssiger Schaumstoffkomponenten in der vorgeformten Folie.

Wesentlich für die erfindungsgemäße Vorrichtung ist somit, daß anstelle der bisher verwendeten sperrigen Masken oder kleinen Masken ohne ausreichenden Schutz nunmehr mit einem komprimierbaren Schaumstoffkörper gearbeitet wird, der in gebrauchsfertigem Zustand den im klinischen Alltag zur Narkoseeinleitung verwendeten Beatmungsmasken stark ähnelt. Dadurch wird die Akzeptanz beim Fachpersonal erhöht. Durch das kleine Packvolumen wird ein ständiges Mitführen gefördert (Schlüsselanhängerprinzip), was insofern vorteilhaft ist, als Wiederbelebungssituationen immer und überall auftreten können. Das Unterbringen einer Beatmungsmaske im Verbandskasten eines Autos ist allgemein unzureichend.

Das Gehäuse kann Sollbruchnähte aufweisen. Ferner kann ein Filter oder ein Rückschlagventil im Dombereich der Maske vorgesehen sein, wodurch auch bei Mund-zu-Maskenbeatmung zusätzlicher Schutz vor Kontamination entsteht.

Bei der erfindungsgemäßen Maske handelt es sich um einen Einmalartikel. Der Schaumstoffkörper kann jedoch als Recyclingmaßnahme nach Trennung einer ihn umhüllenden Folie und nach entsprechender Reinigung erneut in eine neue Folie eingebracht werden.

Ein Ausführungsbeispiel der Erfindung wird unter Bezugnahme auf die beigefügten Zeichnungen rein beispielhaft erläutert. Es zeigen:
- Fig. 1: eine perspektivische Ansicht einer verpackten Beatmungsmaske gemäß der Erfindung; und
- Fig. 2: eine perspektivische Ansicht einer erfindungsgemäßen Beatmungsmaske in ihrem Gebrauchszustand.

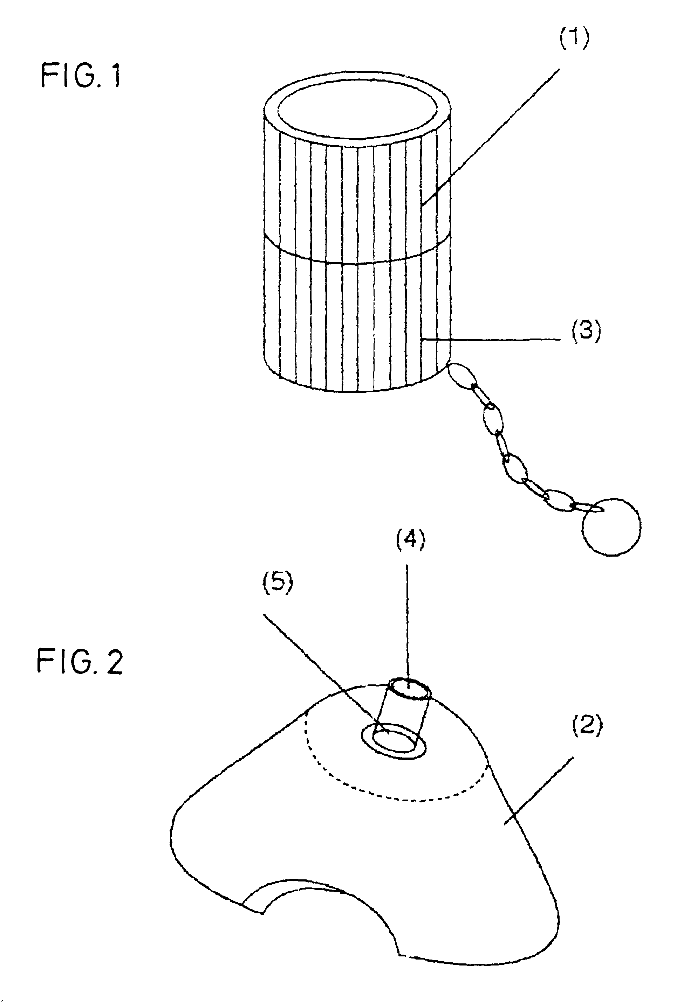

Fig. 1 zeigt ein Gehäuse 1, in dem sich die komprimierte Beatmungsmaske befindet, das in der Praxis beispielsweise eine Grundfläche von 4 cm Durchmesser und eine Höhe von etwa 5 cm aufweist. In diesem Gehäuse, das aus Kunststoffolie besteht, ist der komprimierte Schaumstoffkörper 2 (vgl. Fig. 2) eingeschweißt. Das Gehäuse 1 läßt sich öffnen, indem die Sollbruchnähte 3 geöffnet werden.

Wie aus Fig. 1 zu erkennen ist, verlaufen die Sollbruchnähte 3, welche in die Folie eingearbeitet sind, parallel nebeneinander und an der zylindrischen Mantelfläche des Gehäuses 1. In der Mitte des Gehäuses 1 ist eine weitere Sollbruchnaht vorgesehen, die als Umfangsnaht ausgebildet ist. Auf diese Weise läßt sich das Gehäuse sehr leicht öffnen, indem die beiden Gehäusehälften, die zu beiden Seiten der Umfangsnaht liegen, mit jeweils einer Hand gegriffen und gegeneinander verdreht werden. Hierdurch platzen die Sollbruchnähte 3 des Gehäuses 1 schlagartig auf, so daß sich der Schaumstoffkörper 2 der Beatmungsmaske expandieren kann. Solange sich die Beatmungsmaske in ihrem komprimierten Zustand in dem Gehäuse befindet, läßt sich diese leicht transportieren. Zu diesem Zweck kann auch ein Anhänger oder Schlüsselanhänger (Fig. 1) vorgesehen sein, der an dem Gehäuse befestigt ist.

Fig. 2 zeigt die erfindungsgemäße Beatmungsmaske 2 in ihrem Gebrauchszustand. An der Oberseite der Maske ist eine normierte Öffnung 4 vorgesehen, durch die Atemgas eingeblasen werden kann. Unter dieser Öffnung 4 befindet sich ein Rückschlagventil 5.

Die Form des eingeschweißten Schaumstoffkörpers (Fig. 1) entspricht im Gebrauchszustand (Fig. 2) einem etwa dreieckigen, rund umlaufenden Wulst zum Abdichten auf dem Gesicht (domartige Glocke). So kann die Maske abdichtend und Mund und Nase umschließend auf das Gesicht gepreßt werden, ohne daß Nase oder Mund eingeengt werden. Über einen angeschlossenen Beutel oder über einen Beatmungsschlauch oder mit Hilfe des Mundes kann Luft in die Maske und somit in den Mund und die Nase des Patienten geblasen werden.

## Patentansprüche

1. Verpackte Beatmungsmaske, die durch Volumenzunahme von einem kleineren Packungsvolumen in ihre endgültige Gebrauchsform bringbar ist und die aus einem komprimierbaren, jedoch formstabilen Schaumstoffkörper (2) besteht, der in komprimiertem Zustand in einem Gehäuse (1) aufgenommen ist.

2. Vorrichtung nach Anspruch 1,
**dadurch gekennzeichnet,**
**daß** das Gehäuse (1) Sollbruchstellen aufweist.

3. Vorrichtung nach zumindest einem der vorstehenden Ansprüche, **dadurch gekennzeichnet,**
**daß** die für das Beatmungsgas an der Beatmungsmaske vorgesehene Zuführöffnung (4) normiert ist.

4. Vorrichtung nach zumindest einem der vorstehenden Ansprüche,
**dadurch gekennzeichnet,**
**daß** unterhalb der für das Beatmungsgas vorgesehenen Zuführöffnung (4) ein Filter oder ein Rückschlagventil (5) vorgesehen ist.

5. Vorrichtung nach zumindest einem der vorstehenden Ansprüche,
**dadurch gekennzeichnet,**
**daß** die Form der Beatmungsmaske im Gebrauchszustand einer Narkosemaske entspricht.

## Claims

1. Packed respiratory mask which can be brought by an increase in volume from a smaller packaged volume into its final form of use and which comprises a compressible foam body (2) which is, however, of stable shape and which is received in the compressed state in a housing (1).

2. Apparatus in accordance with claim 1, **characterized in that** the housing (1) has positions (3) of intended rupture.

3. Apparatus in accordance with at least one of the preceding claims, **characterized in that** the supply opening (4) provided at the respiratory mask for the respiratory gas is normed.

4. Apparatus in accordance with at least one of the preceding claims, **characterized in that** a filter or a non-return valve (5) is provided beneath the supply opening (4) provided for the respiratory gas.

5. Apparatus in accordance with at least one of the preceding claims, **characterized in that** the shape of the respiratory mask corresponds in its state of use to a anaesthetic mask.

## Revendications

1. Masque de respiration artificielle emballé, qui peut être amené d'un volume d'emballage plus petit à sa dimension d'utilisation standard définitive par une augmentation de volume et qui est constitué d'un corps en produit alvéolaire (2) comprimable mais stable quant à sa forme et qui est logé à l'état comprimé dans une boîte (1).

2. Dispositif selon la revendication 1,
**caractérisé en ce que**
la boîte (1) comporte des points destinés à la rupture.

3. Dispositif selon au moins l'une des revendications précédentes,
**caractérisé en ce que**
l'orifice d'alimentation (4) prévu pour le gaz de respiration artificielle sur le masque de respiration artificielle est normalisé.

4. Dispositif selon au moins l'une des revendications précédentes,
**caractérisé en ce que**
un filtre ou un clapet de non-retour (5) est prévu en dessous de l'orifice d'alimentation (4) prévu pour le gaz de respiration artificielle.

5. Dispositif selon au moins l'une des revendications précédentes,
**caractérisé en ce que**
la forme du masque de respiration artificielle à l'état prêt à l'utilisation correspond à un masque d'anesthésie.
